# EUROPEAN PATENT APPLICATION

(11) **EP 4 682 174 A1**
(43) Date of publication of application: **21.01.2026**
(21) Application number: 24774078.0
(22) Date of filing: 18.03.2024
(51) Int. Cl.: C07K 19/00, A61K 39/145, A61K 9/51, A61P 31/16, C12N 15/62

(54) **UNIVERSAL INFLUENZA MRNA VACCINE AND USE THEREOF**

(30) Priority: 17.03.2023 CN 202310276580
(71) Applicant: Shanghai Institute Of Biological Products Co., Ltd, Shanghai 200052 (CN)
(72) Inventor: LUO, Jian, Shanghai 200052 (CN); XIONG, Feifei, Shanghai 200052 (CN); SHANG, Baoyuan, Shanghai 200052 (CN); ZHENG, Mei, Shanghai 200052 (CN); LI, Xiuling, Shanghai 200052 (CN)
(74) Representative: karo IP
(86) International application number: PCT/CN2024/082249
(87) International publication number: WO 2024/193513

(57) **Abstract**

Provided is a universal influenza mRNA vaccine. The protein encoded by the universal influenza mRNA vaccine comprises a matrix protein 2 extracellular domain (M2e), a hemagglutinin (HA) stem LAH region and a nucleoprotein (NP) of an influenza A virus. In addition, further provided is the use of the universal influenza mRNA vaccine in a mouse animal model. The universal influenza mRNA vaccine can induce strong humoral immune and cellular immune responses, and can protect animal model mice against various influenza A viruses. The universal influenza mRNA vaccine is safe and effective, and has a rapid production platform.

## Description

### FIELD OF THE INVENTION

The invention relates to the field of biotechnology, and specifically to universal influenza mRNA vaccine. In addition, the invention also relates to the use of the mRNA vaccine.

### BACKGROUND OF THE INVENTION

Influenza virus is an RNA virus that causes influenza in humans or animals, leading to acute upper respiratory tract infections. Through airborne transmission, they are highly likely to trigger periodic large-scale epidemics worldwide (typically occurring each year during winter, spring, and summer). Globally, there are an estimated 1 billion cases annually, among which 3 to 5 million are severe cases, resulting in 290,000 to 650,000 influenza-related respiratory deaths. Since 2022, influenza activity has rebounded both worldwide and in China. The World Health Organization (WHO) recommends annual influenza vaccination, which is the most effective way to prevent influenza. However, due to the frequent antigenic drift and/or antigenic shift of influenza virus hemagglutinin (HA), the antigenicity of vaccine strains does not match that of circulating strains, resulting in the current influenza vaccines ineffectively prevent seasonal and pandemic influenza. It is reported that the effectiveness of seasonal influenza vaccines worldwide is only 10% to 60%. Therefore, there is an urgent need in the market to develop a universal vaccine targeting conserved influenza virus epitopes.

Currently available influenza vaccines in the market mainly include split influenza vaccines, attenuated influenza vaccines, and protein subunit vaccines. Both traditional split influenza vaccines produced based on chicken embryos and those manufactured using cell-based or recombinant protein approaches require a relatively long production time. With the successful application of mRNA technology in COVID-19 vaccines, mRNA technology has garnered significant attention due to its characteristics of high efficiency, safety, and strong immunogenicity. The development of influenza-related mRNA vaccines can bypass a series of complex bioactive processes such as cell fermentation, requiring only *in vitro* synthesis of mRNA, which greatly accelerates the research and development speed and simplifies the production processes. Whether for strain-specific influenza vaccines or universal ones, mRNA technology enables the rapid advancement of candidate influenza vaccines to the clinical trial stage.

### SUMMARY OF THE INVENTION

The purpose of the present invention is to provide an mRNA vaccine encoding universal influenza and its use.

Specifically, one of the technical problems to be solved by the present invention is to provide an mRNA vaccine encoding universal influenza.

The second technical problem to be solved by the present invention is to provide a preparation method for the mRNA vaccine encoding universal influenza.

The third technical problem to be solved by the present invention is to provide a use of the mRNA vaccine encoding universal influenza.

In the first aspect of the present invention, is provided a recombinant protein with a structure represented by the following formula (I) or (II) from the N-terminus to the C-terminus:

S-M-H-N (I),

S-H-M-N (II),

wherein,
each "-" is independently a bond or a linking peptide;
S is absent or a signal peptide;
M is absent, or n tandem influenza virus matrix protein 2 extracellular domains (M2e) or immunogenic fragments thereof;
H is absent, or m tandem hemagglutinin (HA) stem (LAH) regions or immunogenic fragments thereof;
N is nucleoprotein (NP) or an immunogenic fragment thereof;
wherein n is an integer from 1 to 6, and m is an integer from 1 to 6; and
M and H are not absent simultaneously.

In another preferred embodiment, n is 1, 2, 3, or 4.

In another preferred embodiment, m is 1, 2, or 3.

In another preferred embodiment, the signal peptide is selected from the group consisting of: tissue-type plasminogen activator signal peptide, signal peptide of serum immunoglobulin E, or a combination thereof.

In another preferred embodiment, S comprises the amino acid sequence as shown in SEQ ID NO: 11.

In another preferred embodiment, the recombinant protein does not enter the cell nucleus when synthesized in cells.

In another preferred embodiment, the nucleoprotein (NP) is the NP protein from the PR8 strain, or a homologous sequence thereof.

In another preferred embodiment, the NP protein from the PR8 strain is generated by amino acid mutations in one or more nuclear localization signal regions of the NP protein from wild-type PR8 strain.

In another preferred embodiment, the nuclear localization signal region comprises amino acid positions 2-12, 197-215, 339-344 of SEQ ID NO: 10, or a combination thereof.

In another preferred embodiment, the mutation is located at amino acid positions corresponding to positions 6, 7, 212, 213, 214, 215, 341, 342 of SEQ ID NO: 10, or a combination thereof.

In another preferred embodiment, the mutation includes a mutation selected from the group consisting of: K6P, R6P, R212P, K213P, R215P, R341P, V342L, or a combination thereof, wherein the numbers of the amino acid positions correspond to those of SEQ ID NO: 10.

In another preferred embodiment, the amino acid sequence of the nucleoprotein is selected from the group consisting of:
(N1) the amino acid sequence of SEQ ID NO: 10;
(N2) an amino acid sequence obtained by substituting, deleting, altering, or inserting one or more amino acid residues in the sequence of SEQ ID NO: 10, or adding 1 to 30 amino acid residues (preferably 1-10 amino acid residues, more preferably 1-5 amino acid residues) at its N-terminus or C-terminus; the obtained amino acid sequence has ≥85% (preferably ≥90%, more preferably ≥95%, such as ≥96%, ≥97%, ≥98% or ≥99%) sequence identity with the sequence of SEQ ID NO: 10; and the obtained amino acid sequence exhibits equivalent or similar immunogenicity to the sequence in (N1) and does not undergo nuclear localization.

In another preferred embodiment, the M represents n tandem identical or different influenza virus matrix protein 2 extracellular domains (M2e).

In another preferred embodiment, the influenza virus is derived from human influenza, avian influenza, swine influenza, or a combination thereof.

In another preferred embodiment, the influenza virus is an influenza A virus.

In another preferred embodiment, the amino acid sequence of the M2e is selected from the group consisting of:
(M1) the amino acid sequence of any one of SEQ ID NOs: 3-6;
(M2) an amino acid sequence obtained by substituting, deleting, altering, or inserting one or more amino acid residues in the sequence of any one of SEQ ID NOs: 3-6, or adding 1 to 3 amino acid residues (preferably 1-2 amino acid residues, more preferably 1 amino acid residue) at its N-terminus or C-terminus; the obtained amino acid sequence has ≥85% (preferably ≥90%, more preferably ≥95%, such as ≥ 96%, ≥97%, ≥98% or ≥99%) sequence identity with the sequence of any one of SEQ ID NOs: 3-6; and the obtained amino acid sequence exhibits equivalent or similar immunogenicity as the sequence in (M1).

In another preferred embodiment, the M has a structure represented by the following formula (III) from the N-terminus to the C-terminus:

M1-M2-M3-M4 (III) ;

wherein,
each "-" is independently a bond or a linking peptide;
M1, M2, M3, and M4 are each independently absent or M2e or an immunogenic fragment thereof, and M1, M2, M3, and M4 are not absent simultaneously.

In another preferred embodiment, the amino acid sequences of M1, M2, M3, and M4 are each independently selected from the group consisting of: SEQ ID NO: 3, 4, 5, and 6.

In another preferred embodiment, the sequences of M1, M2, M3, and M4 are different from each other.

In another preferred embodiment, M1 has the amino acid sequence of SEQ ID NO: 3; M2 has the amino acid sequence of SEQ ID NO: 4; M3 has the amino acid sequence of SEQ ID NO: 5; and M4 has the amino acid sequence of SEQ ID NO: 6.

In another preferred embodiment, the amino acid sequence of M is shown in SEQ ID NO: 12, or has ≥85% (preferably ≥90%, more preferably ≥95%, e.g., ≥96%, ≥97%, ≥98%, or ≥99%) sequence identity to SEQ ID NO: 12, and exhibits equivalent or similar immunogenicity as SEQ ID NO: 12.

In another preferred embodiment, the H is m tandem identical or different hemagglutinin stem LAH regions.

In another preferred embodiment, the HA stem LAH region includes: the long α-helical region on HA2 of influenza A virus subtypes H1, H3, and H5, or a homologous sequence thereof.

In another preferred embodiment, the amino acid sequence of the HA stem LAH region is selected from the group consisting of:
(L1) the amino acid sequence of any one of SEQ ID NOs: 7-9;
(L2) an amino acid sequence obtained by substituting, deleting, altering, or inserting one or more amino acid residues in the sequence of any one of SEQ ID NOs: 7-9, or adding 1 to 10 amino acid residues (preferably 1-5 amino acid residues, more preferably 1-3 amino acid residues) at its N-terminus or C-terminus; the obtained amino acid sequence has ≥85% (preferably ≥90%, more preferably ≥95%, such as ≥96%, ≥97%, ≥98% or ≥99%) sequence identity with the sequence of any one of SEQ ID NOs: 7-9; and the obtained amino acid sequence has the same or similar immunogenicity as the sequence in (L1).

In another preferred embodiment, the H has a structure represented by the following formula (IV) from the N-terminus to the C-terminus:

H1-H2-H3 (IV) ;

wherein,
each "-" is independently a bond;
H1, H2, and H3 are each independently absent or represent a hemagglutinin stem LAH region or an immunogenic fragment thereof, and H1, H2, and H3 are not absent simultaneously.

In another preferred embodiment, the amino acid sequences of H1, H2, and H3 are each independently selected from the group consisting of: SEQ ID NO: 7, 8, and 9.

In another preferred embodiment, the sequences of H1, H2, and H3 are different from each other.

In another preferred embodiment, H1 has the amino acid sequence of SEQ ID NO: 7; H2 has the amino acid sequence of SEQ ID NO: 8; and H3 has the amino acid sequence of SEQ ID NO: 9.

In another preferred embodiment, the amino acid sequence of H is shown in SEQ ID NO: 13, or has ≥85% (preferably ≥90%, more preferably ≥95%, e.g., ≥96%, ≥97%, ≥98%, or ≥99%) sequence identity to SEQ ID NO: 13, and exhibits equivalent or similar immunogenicity as SEQ ID NO: 13.

In another preferred embodiment, the linking peptide is selected from the group consisting of: GG, GGS, (G3S)4, or a combination thereof.

In another preferred embodiment, the recombinant protein comprises the amino acid sequence as set forth in SEQ ID NO: 1; or an amino acid sequence having ≥85% (preferably ≥90%, more preferably ≥95%, e.g., ≥96%, ≥97%, ≥98%, or ≥99%) sequence identity to SEQ ID NO: 1 and exhibiting equivalent or similar immunogenicity as SEQ ID NO: 1.

In another preferred embodiment, the recombinant protein further comprises an optional tag sequence that assists in expression and/or purification.

In another preferred embodiment, the tag sequence comprises a 6His tag.

In another preferred embodiment, the recombinant protein is a monomer, dimer, or multimer.

In the second aspect of the present invention, provided is a polynucleotide encoding the recombinant protein according to the first aspect of the present invention.

In another preferred embodiment, the polynucleotide is mRNA.

In another preferred embodiment, the mRNA has a structure represented by the following formula (V):

Z1-Z2-Z3-Z4-Z5 (V)

wherein,
each "-" is independently a bond or a linker;
Z1 is absent or represents a 5' capping element;
Z2 is a 5'-UTR element;
Z3 is the coding sequence of the recombinant protein according to claim 1;
Z4 is a 3'-UTR element;
Z5 is absent or represents a poly A tail element.

In another preferred embodiment, the Z1 is selected from m7GpppG cap, 3'-O-methyl-m7GpppG cap, or anti-reverse cap analog.

In another preferred embodiment, the mRNA has the nucleotide sequence of SEQ ID NO: 2.

In another preferred embodiment, the mRNA is natural or modified mRNA.

In another preferred embodiment, the modified mRNA is modified by partially or fully substituting natural uridine with modified uridine.

In another preferred embodiment, the modified mRNA is modified by fully replacing natural uridine with 1-methyl-pseudouridine.

In the third aspect of the present invention, is provided an mRNA vaccine composition, which comprises:
(i) the mRNA according to the second aspect of the present invention;
(ii) a vaccinologically acceptable carrier.

In another preferred embodiment, the carrier is a lipid nanoparticle.

In another preferred embodiment, the mRNA or recombinant protein is encapsulated in the lipid nanoparticle.

In another preferred embodiment, the lipid nanoparticle comprises cationic lipids, neutral phospholipids, steroidal lipids, or polyethylene glycol-lipids.

In another preferred embodiment, the vaccine composition is an injection or an inhalant.

In the fourth aspect of the present invention, is provided an expression vector containing the polynucleotide according to the second aspect of the present invention.

In another preferred embodiment, the vector includes: bacterial plasmids, bacteriophages, yeast plasmids, plant cell viruses, mammalian cell viruses (such as adenoviruses, retroviruses), or other vectors.

In the fifth aspect of the present invention, is provided a host cell containing the expression vector according to the fourth aspect of the present invention.

In another preferred embodiment, the host cell is a mammalian cell.

In the sixth aspect of the present invention, is provided a method for preparing the vaccine composition according to the third aspect of the present invention, comprising the steps of:
(S1) providing the mRNA according to the second aspect of the present invention; and
(S2) mixing the mRNA with lipid nanoparticles to form liposomal nanoparticles encapsulating the mRNA.

In the seventh aspect of the present invention, is provided a use of the recombinant protein according to the first aspect of the present invention, the polynucleotide according to the second aspect of the present invention, the vaccine composition according to the third aspect of the present invention, the expression vector according to the fourth aspect of the present invention, and/or the host cell according to the fifth aspect of the present invention, for preparing a medicament, wherein the medicament is used for:
(a) preventing influenza; and/or
(b) inducing humoral immunity and/or cellular immunity against influenza viruses in mammals.

In another preferred embodiment, the influenza is influenza A.

In another preferred embodiment, the influenza is selected from the group consisting of: human influenza, avian influenza, swine influenza, or a combination thereof.

In another preferred embodiment, the influenza is caused by viruses selected from the group consisting of: H1N1, H3N2, H9N2, or a combination thereof.

In the eighth aspect of the present invention, is provided a method for preventing influenza, which comprises administering to a subject in need thereof the recombinant protein according to the first aspect of the present invention, the polynucleotide according to the second aspect of the present invention, the vaccine composition according to the third aspect of the present invention, the expression vector according to the fourth aspect of the present invention, and/or the host cell according to the fifth aspect of the present invention.

In another preferred embodiment, the subject is a human or a non-human mammal.

In another preferred embodiment, the non-human mammals include: mice, rats, avians, pigs, dogs, cats, rabbits.

In another aspect of the present invention, is provided a use of the universal influenza mRNA for inducing a humoral immune response, inducing a cellular immune response in a model mouse, and/or protecting model mouse against infection by multiple influenza A virus strains.

It should be understood that, within the scope of the present invention, the technical features specifically described above and below (such as the Examples) can be combined with each other, thereby constituting a new or preferred technical solution which needs not be described one by one.

### DESCRIPTION OF THE DRAWINGS

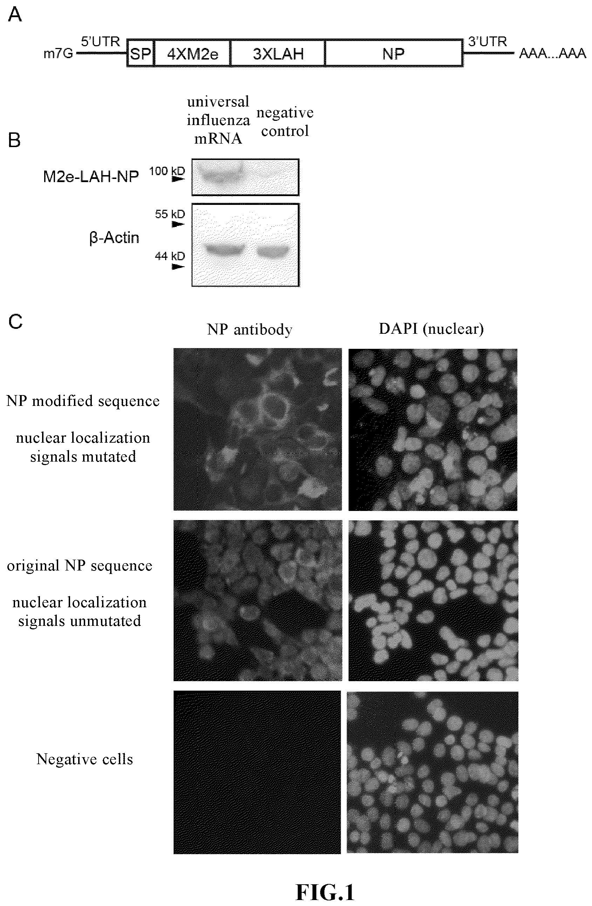

Figure 1 shows the structural schematic diagram and intracellular expression of the universal influenza mRNA vaccine in example 1 of the present invention. Specifically, Figure 1A shows the structural schematic diagram of the universal influenza mRNA vaccine; Figure 1B shows the Western blotting result after transfection of universal influenza mRNA into 293T cells; Figure 1C shows the immunofluorescence result after transfection of universal influenza mRNA into 293T cells. The nuclear localization signal in the NP segment of the mRNA sequence is mutated, enabling the M2e-LAH-NP protein encoded by the mRNA to be present in the cytoplasm, while the unmutated original NP sequence leads the M2e-LAH-NP protein to enter the cell nucleus, which is not conducive for its expression.

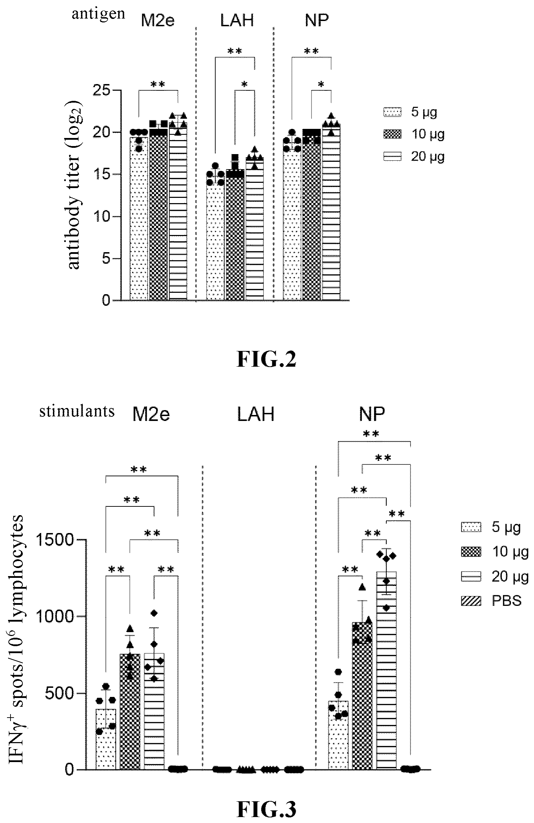

Figure 2 shows the antibody titers in mice after two intramuscular immunizations with different doses of the universal influenza mRNA vaccine in example 2 of the present invention.
Figure 3 shows the number of IFN-γ-secreting splenic lymphocyte spots in mice after two intramuscular immunizations with different doses of the universal influenza mRNA vaccine in embodiment 3 of the present invention.

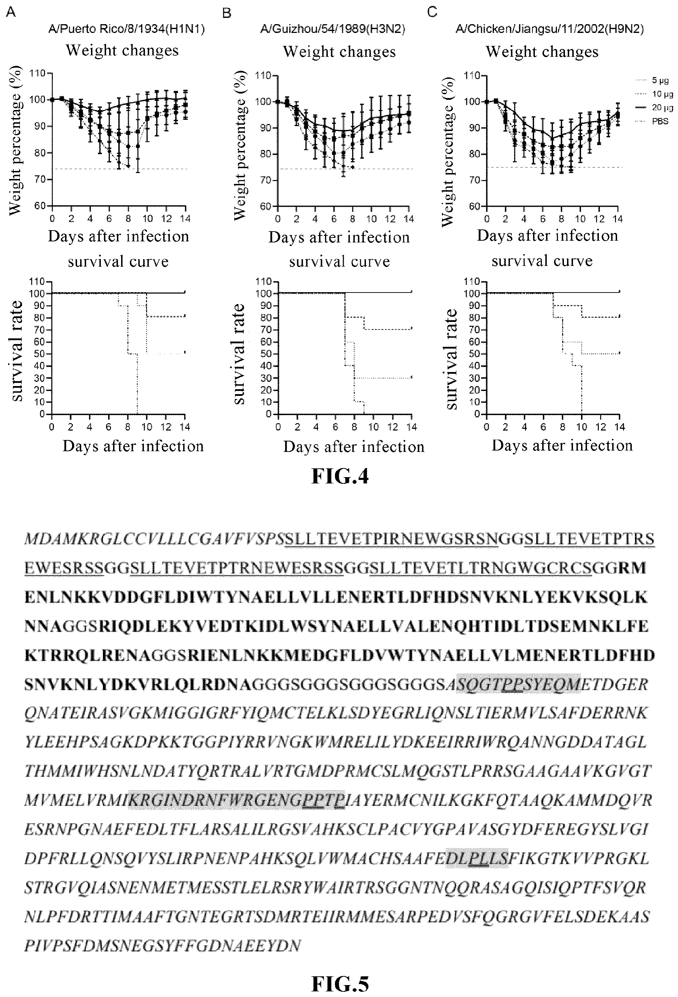

Figure 4 shows the preventive protection experiment of different doses of the universal influenza mRNA vaccine on mice infected with lethal doses of H1N1, H3N2, and H9N2 viruses in example 4 of the present invention. Among them, Figure 4A shows the schematic diagram of body weight loss and the survival curve of mice infected with H1N1 virus; Figure 4B shows the schematic diagram of body weight loss and the survival curve of mice infected with H3N2 virus; Figure 4C shows the schematic diagram of body weight loss and the survival curve of mice infected with H9N2 virus.
Figure 5 shows the representative sequence of the recombinant protein of the present invention, wherein positions 1-23 correspond to the signal peptide, the underlined sequence represents the M2e protein, the bold sequence indicates the LAH protein, the italic sequence denotes the NP protein, the gray-background sequence shows the nuclear localization region of the NP protein, and the double-underlined sequence represents the mutated sites.

### Modes for carrying out the invention

After extensive and intensive research and widely screening, the inventors selected the conserved regions (M2e), the HA stem, and the NP protein of influenza A virus, as target genes for the universal influenza vaccine. M2 is a transmembrane protein on the surface of influenza viruses, with a highly conserved amino acid sequence. In particular, the extracellular region (M2e), which is composed of only 23 amino acid residues at the N-terminal of M2, is a candidate target for universal influenza vaccines. There are slight differences in the M2e sequences among human, swine, and avian influenza viruses. In the present invention, the M2e regions from human, swine, and avian influenza viruses are combined in a tandem manner to cover more influenza virus strains. The HA stem is the main part of influenza virus that mediates membrane fusion. Compared with the HA head region, the sequence and structure of the HA stem are more conserved across different influenza subtypes. Antibodies that can broadly neutralize this domain are considered a potential approach to combat various influenza virus strains. LAH region is one of the most conserved parts within the HA stem. The NP protein is a conserved structural protein with a very low mutation rate during viral evolution and strong cross-immunogenicity, making it a well-studied target for universal influenza vaccines.

In the present invention, the immunogenic and conserved regions of these three influenza viruses are concatenated and prepared in the form of an mRNA-LNP vaccine. In the evaluation of immunogenicity in mouse models, this universal influenza mRNA vaccine shows strong humoral and cellular immune responses, and can effectively prevent and protect mice from attacks by lethal doses of H1N1, H3N2, and H9N2 viruses. This vaccine has promising application prospects as a universal influenza vaccine.

The inventors mutated the nuclear localization signal region of the NP protein, enabling the M2e-LAH-NP protein encoded by the mRNA of the present invention to be present in the cytoplasm without entering the cell nucleus, which facilitates protein expression.

### Terms

To make it easier to understand the present invention, certain technical and terms are specifically defined below. Unless otherwise clearly defined herein, each of the following terms shall have the meaning given below. Other definitions were expounded throughout the application.

The term "about" may refer to a value or composition within an acceptable error range for a particular value or composition as determined by those skilled in the art, which will depend in part on how the value or composition is measured or determined.

As used herein, the terms "having" or "comprising (including)" can be open, semi-closed, and closed. In other words, the term also includes "consisting essentially of" or "consisting of".

As used herein, the terms "host," "subject," and "individual in need" refer to any mammal or non-mammal. Mammals include, but are not limited to, cats, other vertebrates such as rodents, humans, non-human primates. For example, cattle, horses, dogs, pigs, sheep, goats, giraffes, deer, camels, antelopes, rats, mice, hares, and rabbits.

### Recombinant protein

In the present invention, is provided a recombinant protein, which comprises a structure formed by connecting the immunogenic and conserved regions of three influenza viruses in tandem. The recombinant protein exhibits the immunogenicity of the influenza viruses and can induce humoral and cellular immune responses against the influenza viruses.

As used herein, the term "recombinant protein" or "M2e-LAH-NP protein" refers to a recombinant protein comprising antigenic peptides or structural proteins selected from the group consisting of:
(1) The extracellular domain of influenza virus matrix protein 2 (M2e), or an immunogenic fragment thereof;
(2) The LAH region of the hemagglutinin (HA) stem, or an immunogenic fragment thereof;
(3) Nucleoprotein (NP), or an immunogenic fragment thereof.

In a preferred embodiment, the recombinant protein of the present invention is encoded by the mRNA vaccine of the present invention.

M2 is a transmembrane protein on the surface of influenza viruses, with a highly conserved amino acid sequence. In particular, the extracellular region (M2e), which is composed of only 23 amino acid residues at the N-terminal of M2, is a candidate target for universal influenza vaccines. There are slight differences in the M2e sequences among human, swine, and avian influenza viruses. In the present invention, the M2e regions from human, swine, and avian influenza viruses are combined in a tandem manner to cover more influenza virus strains.

In a preferred embodiment, the recombinant protein of the present invention comprises one or more M2e sequences selected from the group consisting of:
SEQ ID NO: 3: SLLTEVETPIRNEWGSRSN;
SEQ ID NO: 4: SLLTEVETPTRSEWESRSS;
SEQ ID NO: 5: SLLTEVETPTRNEWESRSS; and
SEQ ID NO: 6: SLLTEVETLTRNGWGCRCS.

Preferably, the recombinant protein of the present invention comprises a sequence formed by connecting SEQ ID NO: 3-6 in tandem.

The HA stem is the main part of influenza virus that mediates membrane fusion. Compared with the HA head region, the sequence and structure of the HA stem are more conserved across different influenza subtypes. Antibodies that can broadly neutralize this domain are considered a potential approach to combat various influenza virus strains. LAH region is one of the most conserved parts within the HA stem.

In a preferred embodiment, the recombinant protein of the present invention comprises one or more LAH sequences selected from the group consisting of:
SEQ ID NO: 7: 
RMENLNKKVDDGFLDIWTYNAELLVLLENERTLDFHDSNVKNLYEKVKSQLK
NNA;
SEQ ID NO: 8: 
RIQDLEKYVEDTKIDLWSYNAELLVALENQHTIDLTDSEMNKLFEKTRRQLREN
A; and
SEQ ID NO: 9:

RIENLNKKMEDGFLDVWTYNAELLVLMENERTLDFHDSNVKNLYDKVRLQLR
DNA.

Preferably, the recombinant protein of the present invention comprises a sequence formed by connecting SEQ ID NO: 7-9 in tandem.

The NP protein is a conserved structural protein with a very low mutation rate during viral evolution and strong cross-immunogenicity, making it a well-studied target for universal influenza vaccines. The wild-type NP protein contains a nuclear localization signal and tends to enter the cell nucleus after expression, which is unfavorable for vaccine secretion. Through screening, the present invention unexpectedly found that partial mutation of the NP nuclear localization signal enables the mRNA-encoded M2e-LAH-NP protein to be present in the cytoplasm, thereby facilitating its secretion out of the cell.

The fusion protein of the present invention may comprise a signal peptide. The signal peptide sequence can be shown as SEQ ID NO: 11:
MDAMKRGLCCVLLLCGAVFVSPS (SEQ ID NO: 11).

In a preferred embodiment, the recombinant protein of the present invention comprises the NP sequence of SEQ ID NO: 10:

ASQGTPPSYEQMETDGERQNATEIRASVGKMIGGIGRFYIQMCTELKLSDY
EGRLIQNSLTIERMVLSAFDERRNKYLEEHPSAGKDPKKTGGPIYRRVNGKWM
RELILYDKEEIRRIWRQANNGDDATAGLTHMMIWHSNLNDATYQRTRALVRTG
MDPRMCSLMQGSTLPRRSGAAGAAVKGVGTMVMELVRMIKRGINDRNFWRG
ENGPPTPIAYERMCNILKGKFQTAAQKAMMDQVRESRNPGNAEFEDLTFLARS
ALILRGSVAHKSCLPACVYGPAVASGYDFEREGYSLVGIDPFRLLQNSQVYSLIR
PNENPAHKSQLVWMACHSAAFEDLPLLSFIKGTKVVPRGKLSTRGVQIASNEN
METMESSTLELRSRYWAIRTRSGGNTNQQRASAGQISIQPTFSVQRNLPFDRTTI
MAAFTGNTEGRTSDMRTEIIRMMESARPEDVSFQGRGVFELSDEKAASPIVPSF
DMSNEGSYFFGDNAEEYDN(SEQ ID NO: 10).

In the recombinant protein of the present invention and the mRNA encoding it, the sequences of M2e protein, LAH protein, and NP protein can be connected tandemly in any order, provided that the resulting protein can be expressed and secreted out of the cell. In a preferred embodiment, due to the large molecular weight of the NP protein and its possession of a nuclear localization signal, the NP protein is linked to the C-terminus of the recombinant protein of the present invention.

In the present invention, is provided a scheme for sequence design of a universal influenza vaccine. The universal influenza vaccine comprises at least one antigenic peptide or structural protein, including M2e of influenza A virus or an immunogenic fragment thereof, the LAH region of the HA stem or an immunogenic fragment thereof, and NP or an immunogenic fragment thereof. In a preferred embodiment, the recombinant protein of the present invention comprises M2e with amino acid sequences of SEQ ID NO: 3-6, LAH with amino acid sequences of SEQ ID NO: 7-9, and/or NP with amino acid sequences of SEQ ID NO: 10.

In a preferred embodiment, the recombinant protein of the present invention comprising a structure represented by the following formula (I) or (II) from the N-terminus to the C-terminus:

S-M-H-N (I),

S-H-M-N (II),

wherein,
each "-" is independently a bond or a linking peptide;
S is absent or represents a signal peptide;
M is absent, or n tandem influenza virus matrix protein 2 extracellular domains (M2e) or immunogenic fragments thereof, wherein n is an integer from 1 to 6, preferably 1, 2 or 3;
H is absent, or m tandem hemagglutinin (HA) stem (LAH) regions or immunogenic fragments thereof, wherein m is an integer from 1 to 6, preferably 1, 2 or 3;
N is nucleoprotein (NP) or an immunogenic fragment thereof; and
M and H are not absent simultaneously.

Preferably, the amino acid sequence of M is shown in SEQ ID NO: 12, or has ≥85% (preferably ≥90%, more preferably ≥95%, e.g., ≥96%, ≥97%, ≥98%, or ≥99%) sequence identity to SEQ ID NO: 12, and exhibits equivalent or similar immunogenicity as SEQ ID NO: 12.

SLLTEVETPIRNEWGSRSNGGSLLTEVETPTRSEWESRSSGGSLLTEVETPT
RNEWESRSSGGSLLTEVETLTRNGWGCRCS (SEQ ID NO: 12).

Preferably, the amino acid sequence of H is shown in SEQ ID NO: 13, or has ≥85% (preferably ≥90%, more preferably ≥95%, e.g., ≥96%, ≥97%, ≥98%, or ≥99%) sequence identity to SEQ ID NO: 13, and exhibits equivalent or similar immunogenicity as SEQ ID NO: 13.

RMENLNKKVDDGFLDIWTYNAELLVLLENERTLDFHDSNVKNLYEKVKS
QLKNNAGGSRIQDLEKYVEDTKIDLWSYNAELLVALENQHTIDLTDSEMNKLF

EKTRRQLRENAGGSRIENLNKKMEDGFLDVWTYNAELLVLMENERTLDFHDS
NVKNLYDKVRLQLRDNA (SEQ ID NO: 13).

The fusion protein of the present invention may optionally comprise a peptide linker. The size and complexity of the peptide linker may affect the activity of the protein. Generally, the peptide linker should have sufficient length and flexibility to ensure that the two connected proteins have sufficient spatial freedom to exert their functions. In a preferred embodiment of the present invention, the length of the peptide linker is generally 0-15 amino acids, preferably 0-10 amino acids, more preferably 0-5 amino acids, such as GG, GGS, GGGSGGGSGGGSGGGS (SEQ ID NO: 14).

Preferably, the amino acid sequence of the recombinant protein of the present invention is as shown in SEQ ID NO: 1:

MDAMKRGLCCVLLLCGAVFVSPSSLLTEVETPIRNEWGSRSNGGSLLTEVE
TPTRSEWESRSSGGSLLTEVETPTRNEWESRSSGGSLLTEVETLTRNGWGCRCS
GGRMENLNKKVDDGFLDIWTYNAELLVLLENERTLDFHDSNVKNLYEKVKSQ
LKNNAGGSRIQDLEKYVEDTKIDLWSYNAELLVALENQHTIDLTDSEMNKLFE
KTRRQLRENAGGSRIENLNKKMEDGFLDVWTYNAELLVLMENERTLDFHDSN
VKNLYDKVRLQLRDNAGGGSGGGSGGGSGGGSASQGTPPSYEQMETDGERQN
ATEIRASVGKMIGGIGRFYIQMCTELKLSDYEGRLIQNSLTIERMVLSAFDERRN
KYLEEHPSAGKDPKKTGGPIYRRVNGKWMRELILYDKEEIRRIWRQANNGDDA
TAGLTHMMIWHSNLNDATYQRTRALVRTGMDPRMCSLMQGSTLPRRSGAAG
AAVKGVGTMVMELVRMIKRGINDRNFWRGENGPPTPIAYERMCNILKGKFQT
AAQKAMMDQVRESRNPGNAEFEDLTFLARSALILRGSVAHKSCLPACVYGPAV
ASGYDFEREGYSLVGIDPFRLLQNSQVYSLIRPNENPAHKSQLVWMACHSAAFE
DLPLLSFIKGTKVVPRGKLSTRGVQIASNENMETMESSTLELRSRYWAIRTRSGG
NTNQQRASAGQISIQPTFSVQRNLPFDRTTIMAAFTGNTEGRTSDMRTEIIRMME
SARPEDVSFQGRGVFELSDEKAASPIVPSFDMSNEGSYFFGDNAEEYDN (SEQ
ID NO: 1).

### mRNA vaccine

As used herein, the term "mRNA" refers to a type of single-stranded ribonucleic acid that is transcribed from one strand of DNA as a template, carries genetic information, and can direct protein synthesis.

As used herein, the term "vaccine" refers to a composition suitable for application in animals (including humans) that induces an immune response after administration, with a strength sufficient to at least help prevent, ameliorate, or cure clinical diseases caused by microbial infections.

As used herein, the terms "mRNA of the present invention", "mRNA vaccine", "universal influenza mRNA vaccine", and "mRNA sequence of the present invention" are used interchangeably, all referring to the mRNA described in the second aspect of the present application.

In another preferred embodiment, the mRNA has a structure represented by the following formula (V):

Z1-Z2-Z3-Z4-Z5 (V)

wherein,
each "-" is independently a bond or a linker;
Z1 is absent or represents a 5' capping element;
Z2 is a 5'-UTR element;
Z3 is the coding sequence of the recombinant protein according to claim 1;
Z4 is a 3'-UTR element;
Z5 is absent or represents a poly A tail element.

Preferably, the mRNA vaccine of the present invention has the nucleotide sequence of SEQ ID NO: 2:

auggacgccaugaagagaggccucugcugcgugcugcugcugugcggcgccguguucgugagccccagc
ucccuccucacagagguggagacacccauccggaacgaguggggcagcagaagcaacggaggcagccuccucacc
gagguggagacacccaccagauccgagugggagagcagauccagcggaggcagccuccugaccgagguggagac
acccaccagaaacgagugggagagcagauccagcggaggcagccuccugaccgagguggagacacucaccagaaa
cggcuggggcugcagaugcagcggcggacggauggagaacuuaaacaagaagguggacgacggcuuccuggac
aucuggaccuacaacgccgaguuguuagugcuacuggagaacgagcggacccuggacuuccacgacagcaacgu
gaagaaccuguacgagaaggugaagucccaguuaaagaacaacgcuggaggcagccggauccaggaccuggaga
aguacguggaggacaccaagaucgaccuuuggagcuacaacgccgagcuucucguggcucuggagaaccagcac
accaucgaccucaccgacagcgagaugaacaaguuguucgagaagaccagacggcagcugcgggagaacgcugg
aggcagccggaucgagaacuugaacaagaagauggaggacggcuuccucgacguguggaccuacaacgccgagu
uguuggugcucauggagaacgagagaacccucgacuuccacgacuccaacgugaagaaccuuuacgacaaggug
cgguuacagcugagagacaacgcuggcggaggcagcggcggaggcuccggcggaggcagcggcggaggcuccg
ccagccagggcaccccucccagcuacgagcagauggagacagacggcgagcggcagaacgccaccgagaucagag
ccagcgugggcaagaugaucggcggcaucggccgguucuacauccagaugugcaccgagcugaagcucagcgac
uacgagggcagauugauccagaacagcuuaaccaucgagcggauggugcucagcgccuucgacgagagacggaa
caaguaccuggaggagcaccccagcgccggcaaggaccccaagaagaccggcggcccuaucuaccggagagugaa
cggcaaguggaugcgggagcucauccuuuacgacaaggaggagaucagacggaucuggcggcaggccaacaacg
gcgacgacgccaccgcuggccugacccacaugaugaucuggcacagcaacuugaacgacgccaccuaccagcgga
ccagagcccuugugcggaccggcauggacccucggaugugcagccugaugcagggcagcacccucccuagacgg

uccggcgcugccggcgcugccgugaagggcgugggcaccauggugauggaguuggugcggaugaucaagagag
gcaucaacgaccggaacuucuggagaggcgagaacggcccucccacacccaucgccuacgagcggaugugcaaca
uccucaagggcaaguuccagacagccgcccagaaggccaugauggaccaggugcgggagagccggaacccuggc
aacgccgaguucgaggaccucaccuuccuagcccggagcgcccucaucuugcggggcagcguggcccacaagag
cugccugccugccugcguguacggcccugccguggccagcggcuacgacuucgagcgggagggcuacagccua
gugggcaucgaccccuuccggcugcuucagaacagccagguguacucccuaaucagacccaacgagaaccccgcc
cacaagucccagcugguguggauggccugccacagcgccgccuucgaggaccuacccuuauuaagcuucaucaa
gggcaccaagguggugccucggggcaagcuuagcacacggggcgugcagaucgccagcaacgagaacauggaga
caauggaguccagcacccuugagcugcggagccgguacugggccauccggaccagaagcggcggcaacaccaac
cagcagagagccagcgcuggccagaucagcauccagcccaccuucagcgugcagcggaaccuccccuucgaccgg
accaccaucauggccgccuucacaggcaacaccgagggcagaaccuccgacaugcggaccgagaucauccggaug
auggagagcgcaagaccagaggacgugagcuuccagggcagaggcguguucgagcucagcgacgagaaggccgc
cagccccaucgugcccagcuucgacaugagcaacgagggcagcuacuucuucggcgacaacgccgaggaguacg
acaacuga (SEQ ID NO: 2).

The present invention also provides a vaccine composition containing the mRNA vaccine of the present invention. In a preferred embodiment, the vaccine composition of the present invention is provided in the form of an mRNA-LNP vaccine.

As used herein, the term "lipid nanoparticles (LNP)" refers to particles with at least one dimension in the nanoscale, which comprise at least one lipid. In a preferred embodiment, the lipids include but are not limited to neutral phospholipids and polyethylene glycol-lipids. As used herein, the term "neutral phospholipids" refers to uncharged phospholipid molecules that are not phosphoglycerides. As used herein, the term "polyethylene glycol-lipids" refers to molecules comprising a lipid moiety and a polyethylene glycol moiety.

In the present invention, is provided a polynucleotide encoding the mRNA nucleic acid sequence of the present invention, the M2e, LAH and NP protein sequences of the present invention, or the recombinant protein of the present invention.

In the present invention, is also provided a vector containing the polynucleotide of the present invention.

In the present invention, is further provided the use of the universal influenza mRNA vaccine for inducing a humoral immune response, inducing a cellular immune response in a mouse model, and/or protecting animal model mice from infection by various influenza A viruses.

### Vaccine Composition

In the present invention, is also provided a vaccine composition, which contains (i) a pharmaceutically acceptable carrier and (ii) the mRNA according to the second aspect of the present invention.

The pharmaceutical composition can be in any suitable form, depending on the administration method required by the patient. It can be provided in unit dosage form, usually placed in a sealed container, and can be provided as part of a kit. Such kits usually (but not necessarily) contain instructions for use. It can contain multiple the unit dosage forms.

The pharmaceutical composition is suitable for any appropriate administration route, such as injection (including subcutaneous, intradermal, intramuscular, intraperitoneal, microneedle or intravenous injection), inhalation, oral administration, nasal, anal and other routes. The composition can be prepared by any method known in the pharmaceutical field, for example, by mixing the active ingredient with a carrier or excipient under sterile conditions.

### Main advantages of the present invention include:

(a) The recombinant protein of the present invention comprises three conserved and immunogenic regions, which are tandemly arranged in multiple copies to enhance its immunogenicity;
(b) The vaccine of the present invention adopts an efficient and safe mRNA vaccine form to accelerate the research and development efficiency;
(c) In the recombinant protein of the present invention, the nuclear localization region of the NP protein has been mutated to reduce its nuclear localization, which is beneficial to the secretion of the recombinant protein out of the cell;
(d) The universal influenza mRNA vaccine of the present invention shows strong humoral and cellular immune responses in the immunogenicity evaluation of mouse models, and can effectively prevent and protect mice from lethal doses of H1N1, H3N2, and H9N2 virus.

The present invention is further illustrated below with reference to specific embodiments. It should be understood that these embodiments are provided solely for illustrating the invention and should not be construed as limiting the scope thereof. For experimental methods where specific conditions are not indicated in the following examples, they are generally performed under conventional conditions, such as those described in Sambrook et al., Molecular Cloning: A Laboratory Manual (New York: Cold Spring Harbor Laboratory Press, 1989), or according to the conditions suggested by the manufacturer. Unless otherwise specified, percentages and parts are calculated by weight.

### Example 1: Preparation and cellular expression evaluation of universal influenza mRNA vaccine

The structure of the universal influenza mRNA is shown in Figure 1A, where the NP sequence has three nuclear localization signals mutated, enabling the encoded protein to be secreted out of the cell instead of entering the cell nucleus. Plasmids for expression of the universal influenza mRNA were artificially synthesized. Following plasmid extraction, linearization via enzymatic digestion, and purification of the linearized plasmid, a high-purity linearized plasmid template was obtained. After UV quantification, it was stored at -20°C for subsequent use. With the aforementioned linearized plasmid as a template, the universal influenza mRNA was generated in accordance with kit instructions using a T7 enzyme *in vitro* transcription kit, a capping kit, and a purification kit. The concentration of mRNA was determined by UV, and the quality of mRNA was assessed via capillary electrophoresis. The mRNA was stored at -80°C for subsequent use.

The prepared universal influenza mRNA was transfected into 293T cells using Lipofectamine 3000, and the cells were lysed after 48 hours of transfection. The cell lysate was separated by 12% SDS-PAGE, transferred to a membrane by semi-dry transfer, and blocked with 5% skimmed milk-PBS for 1 hour. The blocking solution was discarded, and anti-M2 mouse monoclonal antibody was added as the primary antibody, followed by incubation at room temperature for 1 hour. After the cells were thoroughly washed, donkey anti-mouse IgG-HRP was added as the secondary antibody, and incubated at room temperature for 1 hour. After the cells were thoroughly washed, ECL was added for color development, and images were taken using an ECL imaging system. The exposed membrane was subjected to strip membrane regeneration, then re-blocked. The β-actin antibody was added as the primary antibody, and donkey anti-rabbit-HRP was added as the secondary antibody, followed by ECL color development and subsequent imaging. As shown in Figure 1B, cells transfected with the universal influenza mRNA showed a distinct band around 100 kDa, which was consistent with the position of the target protein, while no such band was observed in untransfected cells, demonstrating that the prepared mRNA can be well expressed in 293T cells. As shown in Figure 1C, the M2e-LAH-NP protein encoded by the mRNA can exist in the cytoplasm and then be secreted out of the cell, whereas the unmutated original NP sequence causes the entry of the M2e-LAH-NP protein into the cell nucleus and thus hinders its expression.

The prepared universal influenza mRNA was encapsulated using a formulation containing the cationic liposome ALC-0315. Cationic lipids, neutral phospholipids, steroidal lipids, and polyethylene glycol-lipids were dissolved and mixed in ethanol at a molar ratio of 46:9:43:2. The total flow rate of the nano-drug preparation equipment was set at 12 mL/min, and encapsulation was performed at a flow rate ratio of 3:1 between the mRNA solution and the lipid mixture solution. Upon completion of encapsulation, ultrafiltration was carried out for solution exchange, resulting in the universal influenza mRNA vaccine (mRNA-LNP formulation).

### Example 2: Evaluation of Humoral Immunity of Universal Influenza mRNA Vaccine in Mouse Model

Female BALB/c mice aged 6-8 weeks were intramuscularly injected with 5 µg, 10 µg, and 20 µg of the universal influenza mRNA-LNP formulation prepared in Example 1 on Day 0 and Day 21, respectively, with PBS as the negative control group. Each group consists of 5 mice. Blood samples were collected on Day 21 post the second immunization (Day 42), and the levels of specific IgG antibodies in serum were detected by enzyme-linked immunosorbent assay (ELISA). Synthetic M2e peptides, synthetic LAH peptides, or NP proteins were used as coating antigens, respectively, and coated onto 96-well microplates at 100 µl per well, followed by incubation at 2-8°C overnight. After blocking with blocking solution, the serum serially diluted 2-fold from 2⁵ to 2²⁴ was added to the 96-well plates at 100 µl per well, and incubated at 37°C for 2 hours before washing. Diluted enzyme-labeled secondary antibody (donkey anti-mouse IgG-HRP) was added, incubated at 37°C for 1 hour, and then thoroughly washed. 100 µl of freshly prepared TMB substrate solution was added and incubated at room temperature for 5-10 minutes, and then 50 µl of 2M H₂SO₄ per well was added to terminate the reaction. The OD value at 450 nm was read using a microplate reader. The mean and standard deviation of all readings in the PBS control group were calculated, with the threshold set as mean + 2×standard deviation. The maximum dilution factor at which readings in other groups exceeded the threshold was defined as the antibody titer of serum samples in that group.

As shown in Figure 2, the titers of specific IgG antibodies against M2e, LAH, or NP in serum after the second immunization all increased with the increase of dosage. The antibody titer against M2e reached 2¹⁹-2²¹, with the titer in the 20 µg group being higher than that in the 5 µg group (p < 0.01), while no significant difference was observed between the 20 µg and 10 µg groups or between the 10 µg and 5 µg groups. The antibody titer against LAH reached 2¹⁵-2¹⁷, with the titer in the 20 µg group being higher than that in the 5 µg group (p < 0.01) and the 10 µg group (p < 0.05), while no significant difference was found between the 10 µg and 5 µg groups. The antibody titer against NP reached 2¹⁸-2²¹, with the titer in the 20 µg group being higher than that in the 5 µg group (p < 0.01) and the 10 µg group (p < 0.05), while no significant difference was noted between the 10 µg and 5 µg groups.

### Example 3 Evaluation of Cellular Immunity of Universal Influenza mRNA Vaccine in Mouse Model

Spleen tissues were collected from mice in each group of Example 2 on Day 21 after the second immunization (Day 42) for lymphocyte isolation. The proportion of splenic lymphocytes secreting IFN-γ cytokines was detected by enzyme-linked immunospot assay (ELISpot). After blood collection via eyeball extraction, mice were euthanized by cervical dislocation, immersed in 75% ethanol, and then placed in a biosafety cabinet. Spleens were removed using sterile surgical instruments. The spleens were ground with lymphocyte separation medium until no intact tissue blocks remained, filtered through a 40 µm nylon cell strainer into 15 mL centrifuge tubes, and supplemented with lymphocyte separation medium to a total volume of 5 mL. PRMI 1640 medium was slowly added along the tube wall to approximately 7 mL, maintaining a distinct interface between the upper medium and the lower lymphocyte separation medium. Centrifugation was performed at 800 g for 30 minutes at room temperature to allow lymphocytes to aggregate at the interface. Lymphocytes were aspirated and transferred to 15 mL centrifuge tubes containing 1640 medium, and centrifuged at 250 g for 10 minutes at room temperature for cell washing. After centrifugation, the supernatant was discarded, and lymphocytes were resuspended in 1 mL of medium. Cell counting was performed to record viable cell density and viability. Based on the counting results, lymphocytes were diluted to 5×10⁶ cells/mL with medium for subsequent use. The enzyme-linked immunospot assay was performed using the Mouse IFN-γ ELISpotPlus kit (HRP) from Mabtech, following the manufacturer's instructions. 100 µL of prepared lymphocytes (5×10⁶ cells/mL) was added to each well of the ELISpot plate, with 3 replicate wells per sample. Synthetic M2e peptides, synthetic LAH peptides, or NP proteins were used as stimulants, respectively. Positive and negative control wells were set simultaneously: PHA positive stimulant was added to positive control wells, and medium was added to negative control wells. The plate was placed in a humidified chamber and incubated in a 37°C, 5% CO₂ constant temperature incubator for 12-48 hours. The ELISpot plate was removed, the culture medium was discarded, and the plate was washed 5 times with sterile PBS. The primary antibody from the kit was diluted to 1 µg/mL with sterile PBS containing 0.5% FBS, mixed thoroughly, and 100 µL was added to each well, followed by incubation at room temperature for 2 hours. The primary antibody was discarded, and the plate was washed 5 times with sterile PBS. The enzyme-labeled secondary antibody from the kit was diluted at a volume ratio of 1:1000 with sterile PBS containing 0.5% FBS, mixed thoroughly, and 100 µL was added to each well, followed by incubation at room temperature for 1 hour. The secondary antibody was discarded, and the plate was washed 5 times with sterile PBS. After the plate was drained, 100 µL of TMB chromogenic solution from the kit was added to each well until distinct spots appeared. The plate was rinsed with sterile pure water to stop chromogenesis, dried in the dark, and read using an ELISPOT plate reader.

As shown in Figure 3, after the second immunization, both M2e and NP as stimulants induced a high number of splenic lymphocytes secreting IFN-γ cytokines, with the number of induced spots increasing with the dosage. Due to the absence of T-cell epitopes, the LAH sequence barely induced splenic lymphocytes secreting IFN-γ cytokines.

### Example 4 Evaluation of Preventive Efficacy of Universal Influenza mRNA Vaccine in Mice

Female BALB/c mice aged 6-8 weeks were intramuscularly injected with 5 µg, 10 µg, and 20 µg of the universal influenza mRNA-LNP formulation prepared in Example 1 on Day 0 and Day 21, respectively, with PBS as the negative control group. On Day 21 after the second immunization (Day 42), 20 µL of 5×LD₅₀ Jiaxing influenza viruses of subtypes H1N1 (A/Puerto Rico/8/1934), H3N2 (A/Guizhou/54/1989), and H9N2 (A/Chicken/Jiangsu/11/2002) were administered to the mice via nasal drip, with 10 mice in each group. The survival and weight loss of the mice were monitored daily until Day 14 post-infection.

As shown in Figure 4, mice in the PBS control group developed obvious flu-like symptoms 3-4 days after infection with H1N1, H3N2, or H9N2 virus, and all died on Day 9 or 10 post-infection. In contrast, mice immunized with the universal influenza mRNA vaccine achieved partial or full protection. As shown in Figure 4A, against H1N1 virus infection, the protection rates of 5 µg, 10 µg, and 20 µg universal influenza mRNA vaccines in mice were 50%, 80%, and 100%, respectively. Mice in the 20 µg group began to regain weight on Day 5 post-infection, while those in the 10 µg and 5 µg groups started to regain weight on Day 8. As shown in Figure 4B, against H3N2 virus infection, the protection rates of 5 µg, 10 µg, and 20 µg universal influenza mRNA vaccines in mice were 30%, 70%, and 100%, respectively. Mice in all three immunized groups began to regain weight on Day 7-8. As shown in Figure 4C, against H9N2 virus infection, the protection rates of mice immunized with 5 µg, 10 µg, and 20 µg universal influenza mRNA vaccines were 50%, 80%, and 100%, respectively. Mice in all three immunized groups began to regain weight on Day 7-8. In summary, two immunizations with 20 µg of the universal influenza mRNA-LNP vaccine provided complete protection against lethal challenges with influenza A viruses of H1N1, H3N2, and H9N2 subtypes in mice.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A recombinant protein wih a structure represented by the following formula (I) or (II) from the N-terminus to the C-terminus:
S-M-H-N (I),
S-H-M-N (II),
wherein,
each "-" is independently a bond or a linking peptide;
S is absent or a signal peptide;
M is absent, or n tandem influenza virus matrix protein 2 extracellular domains (M2e) or immunogenic fragments thereof;
H is absent, or m tandem hemagglutinin (HA) stem (LAH) regions or immunogenic fragments thereof;
N is nucleoprotein (NP) or an immunogenic fragment thereof;
wherein n is an integer from 1 to 6, and m is an integer from 1 to 6; and
M and H are not absent simultaneously.

2. The recombinant protein of claim 1, wherein the nucleoprotein (NP) is generated by amino acid mutations in one or more nuclear localization signal regions of the NP protein from wild-type PR8 strain, and the nuclear localization signal region comprises amino acid positions 2-12, 197-215, 339-344 of SEQ ID NO: 10, or a combination thereof.

3. The recombinant protein of claim 2, wherein the mutation is located at the amino acid positions corresponding to positions 6, 7, 212, 213, 214, 215, 341, 342 of SEQ ID NO: 10, or a combination thereof.

4. The recombinant protein of claim 1, wherein the amino acid sequence of the nucleoprotein is selected from the group consisting of:
(N1) the amino acid sequence of SEQ ID NO: 10;
(N2) an amino acid sequence obtained by substituting, deleting, altering, or inserting one or more amino acid residues in the sequence of SEQ ID NO: 10, or adding 1 to 30 amino acid residues (preferably 1-10 amino acid residues, more preferably 1-5 amino acid residues) at its N-terminus or C-terminus; the obtained amino acid sequence has ≥85% (preferably ≥90%, more preferably ≥95%, such as ≥96%, ≥97%, ≥98% or ≥99%) sequence identity with the sequence of SEQ ID NO: 10; and the obtained amino acid sequence exhibits equivalent or similar immunogenicity to the sequence in (N1) and does not undergo nuclear localization.

5. The recombinant protein of claim 1, wherein the M has a structure represented by the following formula (III) from the N-terminus to the C-terminus:
M1-M2-M3-M4 (III) ;
wherein,
each "-" is independently a bond or a linking peptide;
M1, M2, M3, and M4 are each independently absent or M2e or an immunogenic fragment thereof, and M1, M2, M3, and M4 are not absent simultaneously.

6. The recombinant protein of claim 5, wherein the amino acid sequences of M1, M2, M3, and M4 are each independently selected from the group consisting of: SEQ ID NO: 3, 4, 5, and 6.

7. The recombinant protein of claim 5, wherein the amino acid sequence of M is shown in SEQ ID NO: 12, or has ≥85% (preferably ≥90%, more preferably ≥95%, e.g., ≥96%, ≥97%, ≥98%, or ≥99%) sequence identity to SEQ ID NO: 12, and exhibits equivalent or similar immunogenicity as SEQ ID NO: 12.

8. The recombinant protein of claim 1, wherein the H has a structure represented by the following formula (IV) from the N-terminus to the C-terminus:
H1-H2-H3 (IV) ;
wherein,
each "-" is independently a bond or a linking peptide;
H1, H2, and H3 are each independently absent or represent a hemagglutinin stem LAH region or an immunogenic fragment thereof, and H1, H2, and H3 are not absent simultaneously.

9. The recombinant protein of claim 8, wherein the amino acid sequences of H1, H2, and H3 are each independently selected from the group consisting of: SEQ ID NO: 7, 8, and 9.

10. The recombinant protein of claim 8, wherein the amino acid sequence of H is shown in SEQ ID NO: 13, or has ≥85% (preferably ≥90%, more preferably ≥95%, e.g., ≥96%, ≥97%, ≥98%, or ≥99%) sequence identity to SEQ ID NO: 13, and exhibits equivalent or similar immunogenicity as SEQ ID NO: 13.

11. The recombinant protein of claim 1, wherein the recombinant protein comprises the amino acid sequence as set forth in SEQ ID NO: 1; or an amino acid sequence having ≥85% (preferably ≥90%, more preferably ≥95%, e.g., ≥96%, ≥97%, ≥98%, or ≥99%) sequence identity to SEQ ID NO: 1 and exhibiting equivalent or similar immunogenicity as SEQ ID NO: 1.

12. A polynucleotide encoding the recombinant protein of claim 1.

13. The polynucleotide of claim 12, wherein the polynucleotide is an mRNA having the nucleotide sequence of SEQ ID NO: 2.

14. An mRNA vaccine composition, which comprises:
(i) the polynucleotide of claim 12, wherein the polynucleotide is mRNA;
(ii) a vaccinologically acceptable carrier.

15. The vaccine composition of claim 14, wherein the vaccinologically acceptable carrier is a lipid nanoparticle.
